# EUROPEAN PATENT APPLICATION

(11) **EP 4 030 440 A1**
(43) Date of publication of application: **20.07.2022**
(21) Application number: 20863750.4
(22) Date of filing: 09.09.2020
(51) Int. Cl.: G16H 50/30

(54) **INFORMATION PROCESSING SYSTEM AND PROGRAM**

(30) Priority: 09.09.2019 JP 2019163537
(71) Applicant: Aiwell Inc., Tokyo 102-0084 (JP); Tokyo Institute of Technology, Tokyo 152-8550 (JP)
(72) Inventor: HAYASHI, Nobuhiro, Tokyo 152-8550 (JP)
(74) Representative: Strehl Schübel-Hopf & Partner
(86) International application number: PCT/JP2020/034061
(87) International publication number: WO 2021/049510

(57) **Abstract**

An information processing system includes: an obtaining unit obtaining state information about a state of a living body and distribution information about distribution of physical quantity of biomolecules; and an estimation unit estimating a future state of a target living body based on a two-dimensional electrophoresis image related to the target living body, other two-dimensional electrophoresis image different from the two-dimensional electrophoresis image, and state information related to a particular living body having biomolecules with distribution specified from the other two-dimensional electrophoresis image.

## Description

### Technical Field

The present invention relates to an information processing system and a program.

### Background Art

Patent Document 1 discloses a method for predicting the progression of a pre-cancerous lesion including determining the expression of aPKCλ/τ protein in a sample separated from a subject. By the method, it is predicted that the possibility of the exacerbation of the pre-cancerous lesion is high or the possibility of the amelioration of the pre-cancerous lesion is low when the expression amount of aPKCλ/τ protein is high and/or when aPKCλ/τ protein is localized in the nucleus.

### Citation List

### Patent Literature

Patent Document 1: International Publication No. WO2015/119132

### Summary of Invention

### Technical Problem

There is a technology that predicts the future health state of one human being based on results of comparing information about biomolecules, such as proteins, obtained from the one human being with statistical information about biomolecules. However, estimation of a future state of one living body based on a state of a particular living body has not been performed.

An object of the present invention is to estimate a future state of one living body based on a state of a particular living body.

### Solution to Problem

An invention described in claim 1 provides an information processing system including: an obtaining unit obtaining state information about a state of a living body and distribution information about distribution of physical quantity of biomolecules; and an estimation unit estimating a future state of one living body based on the distribution information related to the one living body, other distribution information different from the distribution information, and the state information related to a particular living body having biomolecules with the distribution specified from the other distribution information.

An invention described in claim 2 provides the information processing system according to claim 1, further including: a life information obtaining unit obtaining life information related to life of a living body; and a determination unit determining contents to be proposed related to life of the one living body based on the state of the one living body estimated by the estimation unit and the life information obtained by the life information obtaining unit.

An invention described in claim 3 provides the information processing system according to claim 2, wherein the estimation unit estimates a future state of the one living body if the contents determined by the determination unit are performed.

An invention described in claim 4 provides the information processing system according to claim 1, wherein the estimation unit estimates time when the one living body reaches the state.

An invention described in claim 5 provides the information processing system according to claim 4, wherein the obtaining unit obtains plural pieces of distribution information about the distribution at different points in time for the one living body, and the estimation unit estimates the time based on change in the distribution of biomolecules specified from at least two pieces of distribution information from among the plural pieces of distribution information.

An invention described in claim 6 provides the information processing system according to claim 1, wherein the obtaining unit obtains plural pieces of distribution information about the distribution at different points in time for the one living body, and plural pieces of state information about a state of the one living body at different points in time, the other distribution information is the distribution information related to the one living body, and the estimation unit extracts, in accordance with the distribution information extracted as the other distribution information from among the plural pieces of distribution information, some of the pieces of state information from among the plural pieces of state information, and estimates a future state of the one living body by the extracted state information.

An invention described in claim 7 provides an information processing system including: an obtaining unit obtaining distribution information about a distribution of physical quantity of biomolecules in a living body; a specifying unit specifying, in the information obtained by the obtaining unit, other distribution information having a predetermined relation with the distribution information related to one living body; and a provision unit providing information based on a state of a particular living body after a point in time at which biomolecules in the particular living body having biomolecules with the distribution related to the other distribution information specified by the specifying unit showed the distribution.

An invention described in claim 8 provides a program causing a computer to implement: an obtaining function obtaining state information about a state of a living body and distribution information about a distribution of physical quantity of biomolecules; and an estimation function estimating a future state of one living body based on the distribution information related to the one living body, other distribution information different from the distribution information, and the state information related to a particular living body having biomolecules with the distribution specified from the other distribution information.

### Advantageous Effects of Invention

According to the present invention, it is possible to estimate a future state of one living body based on a state of a particular living body.

### Brief Description of Drawings

FIG. 1 is a diagram showing an overall configuration example of a living body state specifying system;
FIG. 2 is a diagram showing a hardware configuration example of a server and a terminal;
FIG. 3 is a diagram showing a functional configuration example of the server;
FIGS. 4A and 4B are diagrams showing examples of a living body information management table;
FIG. 5 is a flowchart showing a flow of state specifying processing;
FIG. 6 is a diagram showing an example of specifying of a state by the state specifying processing;
FIG. 7 is a flowchart showing a flow of performed state specifying processing;
FIG. 8 is a diagram showing an example of specifying of a state by the performed state specifying processing;
FIG. 9 is a flowchart showing a flow of time specifying processing; and
FIG. 10 is a diagram showing an example of specifying of time by the time specifying processing.

### Description of Embodiment

Hereinafter, an exemplary embodiment according to the present invention will be described in detail with reference to attached drawings.

### <Configuration of living body state specifying system>

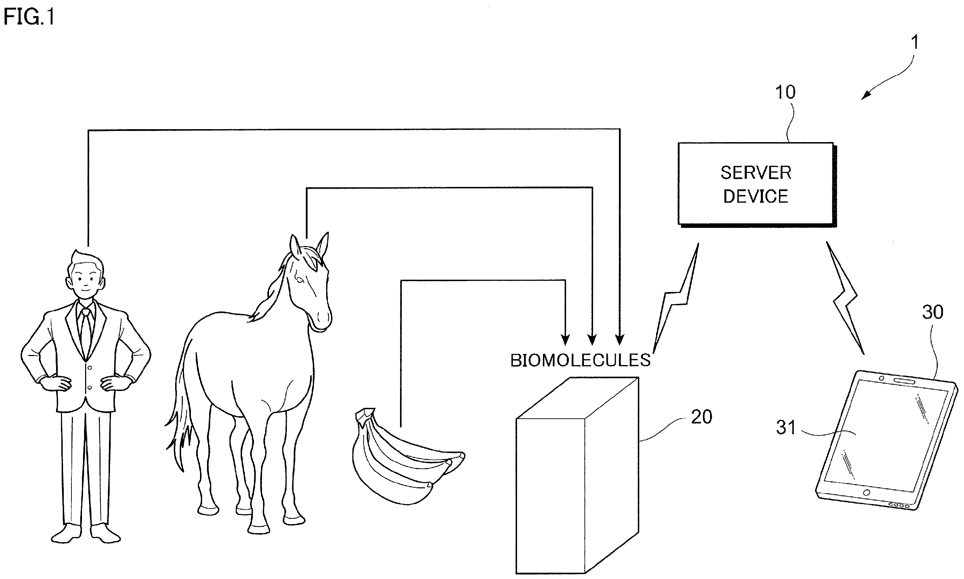

FIG. 1 is a diagram showing an overall configuration example of a living body state specifying system 1 related to the exemplary embodiment.

The living body state specifying system 1 related to the exemplary embodiment is a system that specifies a potential future state of a living body. Examples of the living body include animals, plants, and microorganisms. Examples of the animal include land animals and aquatic animals. In addition, the animals also include human beings. The plants also include agricultural crops.

Examples of the state of the living body include: a health state of a living body; a state about beauty of a living body; a physical state of a living body when the living body is an animal; a state of a brain of a living body when the living body is an animal; a state of quality of a living body when the living body is food; and a state of growth of a living body when the living body is a plant or a microorganism. Examples of the living body that is food include agricultural crops and animals. Examples of the health state of the living body include a state about injury or disease in a living body and a state about fatigue in a living body. In addition, examples of the physical state of the living body include: a state about height of a living body; a state about weight of a living body; a state about a body fat percentage of a living body; and a state about a muscle mass of a living body. Moreover, examples of the state of the brain of the living body include a state about memory of a living body and a state about an ability to think of a living body. Examples of the state of quality of the living body include a state about taste of a living body. In addition, examples of the state of growth of the living body include: a state about the size of each part or the whole of a living body; a state about colors of a living body; a state about a smell of a living body; a state about withering of a living body, and a state about bacteria or viruses in a living body.

The living body state specifying system 1, as an example of an information processing system, includes a server device 10, a biomolecule analysis device 20, and a terminal 30. The server device 10 is connected to the biomolecule analysis device 20 and the terminal 30 via a network.

The server device 10 specifies a potential future state of one living body. More specifically, the server device 10 obtains information about the state of the living body and information about distribution of physical quantities of biomolecules. Examples of the biomolecules include proteins and amino acids. Examples of the physical quantities include charge quantities and molecular weights. Hereinafter, the information about the state of the living body is referred to as state information. In addition, the information about the distribution of physical quantities of biomolecules is hereinafter referred to as distribution information. The server device 10 extracts the distribution information indicating the distribution of biomolecules close to the distribution of biomolecules indicated in the distribution information related to one living body. Then, based on the state information related to a particular living body having the biomolecules indicated in the extracted distribution information, a potential future state of one living body is specified. In other words, in the exemplary embodiment, on the basis that the distribution of physical quantities of biomolecules corresponds to the state of a living body, the potential future state of one living body is specified from the distribution of physical quantities of biomolecules in one living body. Further, the server device 10 provides the terminal 30 with information about the specified state.

The server device 10 is implemented by, for example, a computer. The server device 10 may be configured with a single computer, or may be implemented by distributed processing using plural computers. In addition, the server device 10 may also be implemented on virtual hardware provided by cloud computing.

The biomolecule analysis device 20 analyzes biomolecules obtained from a living body. More specifically, the biomolecule analysis device 20 analyzes the biomolecules, to thereby detect the distribution of physical quantities of biomolecules. As the biomolecule analysis device 20, for example, a two-dimensional electrophoresis device is used. The two-dimensional electrophoresis device separates proteins as an example of biomolecules by a molecular weight and also separates the proteins by a charge quantity. The two-dimensional electrophoresis device also creates a two-dimensional electrophoresis image showing the distribution of the molecular weight and the charge quantity of the proteins. In the two-dimensional electrophoresis image, for example, in a two-dimensional orthogonal coordinate system where the distribution of the charge quantity of biomolecules is shown on the x-axis and the distribution of the molecular weight of biomolecules is shown on the y-axis, the positions where the pixels composing the image representing the biomolecules are disposed are defined. In other words, in a two-dimensional electrophoresis image, information indicating whether or not the biomolecules are located is written for coordinates of each pixel. The two-dimensional electrophoresis image is perceived as an example of the distribution information. In addition, the two-dimensional electrophoresis image may include information indicating the expression amount of biomolecules. In other words, the "distribution of physical quantities of biomolecules" includes not only the "distribution of whether or not the biomolecules are located" but also the "distribution of the expression amount of biomolecules." The information indicating the expression amount of biomolecules in the two-dimensional electrophoresis image may be displayed, for example, by shades of an image showing the biomolecules.

Moreover, as the biomolecule analysis device 20, for example, a biosensor may be used. The biosensor identifies biomolecules in a living body. In addition, the biosensor also separates proteins in the identified biomolecules by a molecular weight and by a charge quantity, to thereby create an image showing the distribution of the molecular weight and the distribution of the charge quantity of the proteins. Therefore, the image created by use of the biosensor is also perceived as the distribution information. Further, the image created by the biosensor may include information indicating the expression amount of biomolecules.

In the exemplary embodiment, the biomolecule analysis device 20 is used to analyze biomolecules and generate distribution information for each living body. In addition, the biomolecule analysis device 20 is used to obtain biomolecules on a regular basis for each living body and create the distribution information related to the collected biomolecules. Thus, for each living body, plural pieces of distribution information are created, each of which shows the distribution of biomolecules at each point in time in a time series.

When the distribution information is created, the biomolecule analysis device 20 transmits the created distribution information to the server device 10 along with identification information that identifies the living body having the biomolecules indicated by the distribution information. Note that the living body "having" the biomolecules includes not only the living body having the target biomolecules, but also the living body that had the target biomolecules in the past, although the living body does not have the target biomolecules because the target biomolecules were collected from the living body.

Note that the biomolecule analysis device 20 is not limited to those analyze the biomolecules collected from the living bodies. For example, if the biomolecule analysis device 20 can analyze the biomolecules in the living body and create the distribution information without collecting the biomolecules from the living body, such a biomolecule analysis device 20 may be used.

The terminal 30 includes a display part 31 that displays information. When the terminal 30 obtains information from the server device 10, the terminal 30 displays the information on the display part 31.

The terminal 30 is implemented by, for example, a computer, a tablet information terminal, or other information processing devices. The terminal 30 may be, for example, a smartphone. In other words, the terminal 30 may be any type of terminal.

The type of network used to connect the server device 10, the biomolecule analysis device 20, and the terminal 30 is not particularly limited as long as data can be transmitted and received, and the network may be, for example, the Internet, LAN (Local Area Network), WAN (Wide Area Network), or others. The communication line used to transmit and receive data may be wired or wireless. In addition, there may be a configuration that connects devices via plural networks and communication lines.

### <Hardware configuration example>

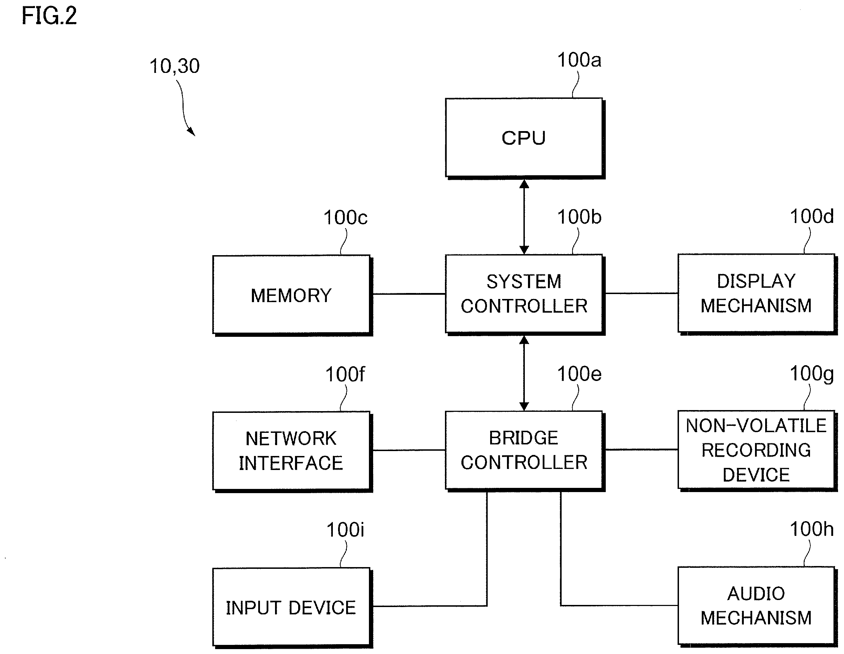

FIG. 2 is a diagram showing a hardware configuration example of the server device 10 and the terminal 30.

As shown in FIG. 2, the server device 10 and the terminal 30 include a central processing unit (CPU) 100a, which is an arithmetic unit, and a memory 100c, which is a main storage unit. Moreover, each device also includes, as external devices, a non-volatile recording device 100g, a network interface 100f, a display mechanism 100d, an audio mechanism 100h, and an input device 100i, such as a keyboard and a mouse.

The memory 100c and the display mechanism 100d are connected to the CPU 100a via a system controller 100b. In addition, the network interface 100f, the non-volatile recording device 100g, the audio mechanism 100h, and the input device 100i are connected to the system controller 100b via a bridge controller 100e. Each constituent is connected by various types of buses, such as a system bus and an input-output bus.

The non-volatile recording device 100g stores a program for implementing each function. The program is then loaded into the memory 100c, and the processing based on the program is performed by the CPU 100a, to thereby implement various functions. Examples of the non-volatile storage device 100g include semiconductor memories, such as solid state drives (SSDs) or magnetic disk devices, such as hard disk drives (HDDs).

### <Functional configuration of server device>

Next, a functional configuration of the server device 10 will be described.

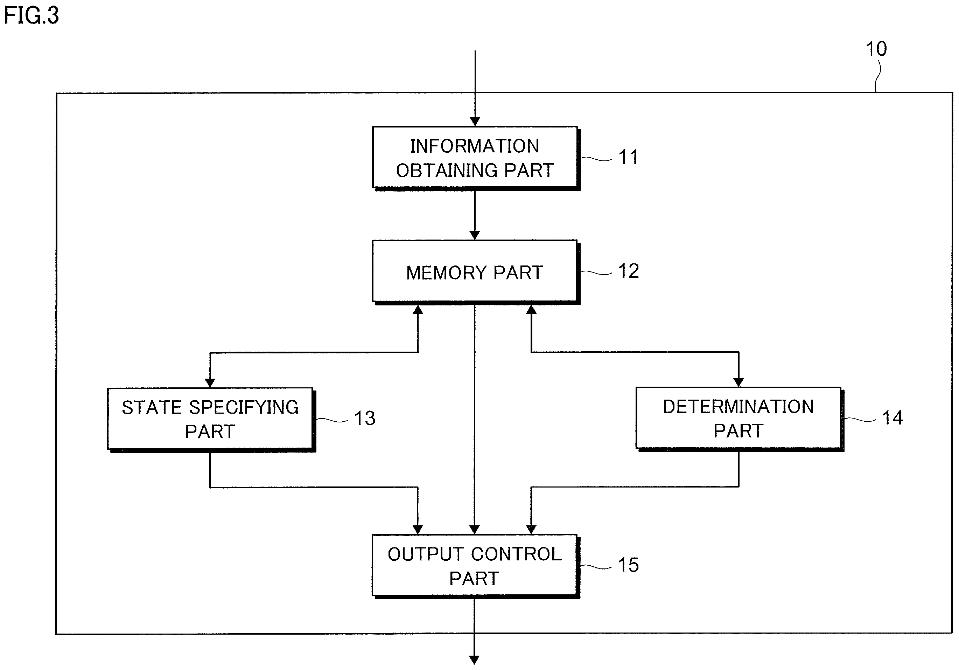

FIG. 3 is a diagram showing a functional configuration example of the server device 10.

The server device 10 includes: an information obtaining part 11 that obtains information; a memory part 12 that stores the information; a state specifying part 13 that specifies a potential future state of a living body; a determination part 14 that determines contents to be proposed regarding life of the living body; and an output control part 15 that outputs the information.

The information obtaining part 11, as an example of an obtaining unit, obtains the distribution information from the biomolecule analysis device 20. The information obtaining part 11 also obtains the state information. For example, the user inputs information indicating a state of a living body to the terminal 30. Here, the information inputted to the terminal 30 is, for example, information indicating a state of the living body when the biomolecules indicated in one piece of distribution information were collected from a living body. The information is inputted to the terminal 30, and thereby the state information corresponding to one piece of distribution information is created by the terminal 30. Note that the state information may be created by the server device 10.

In the exemplary embodiment, the state information is created for each living body. In addition, in the exemplary embodiment, the state information is created on a regular basis for each living body. Thus, for each living body, plural pieces of state information are created, each of which shows the state of the living body at each point in time in a time series.

When the information obtaining part 11 obtains the state information from the terminal 30, the information obtaining part 11 keeps the obtained state information in association with the distribution information corresponding to the state information.

The information obtaining part 11 also obtains information about life of the living body. Examples of the information about life of the living body may include: information about the environment in a place where the living body lives; information about sleep of the living body in the case where the living body is an animal; information about exercise or training that the living body has performed in the case where the living body is an animal; information about meals and nutrition that the living body has taken; and information about products and services that the living body has adopted in the case where the living body is a human being. The information about life of the living body is hereinafter referred to as life information. For example, the user inputs information indicating life of a living body to the terminal 30. Here, the information inputted to the terminal 30 is, for example, information indicating life of a living body when the living body is in a state indicated in one piece of state information. The information is inputted to the terminal 30, and thereby the life information corresponding to one piece of state information is created by the terminal 30. Note that the life information may be created by the server device 10.

In the exemplary embodiment, the life information is created for each living body. In addition, in the exemplary embodiment, the life information is created on a regular basis for each living body. Thus, for each living body, plural pieces of life information are created, each of which shows life of the living body at each point in time in a time series.

When the information obtaining part 11 obtains the life information from the terminal 30, the information obtaining part 11 associates the obtained life information with the state information corresponding to the life information. The distribution information, the state information, and the life information associated with one another are then transmitted to the memory part 12. Note that the information obtaining part 11 can also be perceived as a life information obtaining unit that obtains the life information.

The memory part 12 stores the distribution information, the state information, and the life information transmitted from the information obtaining part 11. Note that the distribution information, the state information, and the life information transmitted from the information obtaining part 11 are associated with one another and stored in the memory part 12. The stored contents of the memory part 12 will be described in detail later.

The state specifying part 13, as an example of an estimation unit, regards one living body as a target and specifies a potential future state of a living body to be the target. The living body to be the target, whose state is specified by the state specifying part 13, is hereinafter referred to as the target living body.

The state specifying part 13, first, compares distribution of physical quantity of biomolecules specified based on distribution information related to the target living body with distribution of physical quantity of biomolecules specified based on distribution information other than the distribution information related to the target living body using the distribution information stored in the memory part 12. The distribution information related to the target living body used for the comparison is, for example, distribution information indicating distribution of biomolecules recently collected from the target living body. In addition, the distribution of physical quantity of biomolecules specified based on the distribution information related to the target living body is hereinafter referred to as the target distribution. Based on the results of comparison of the distribution information, the state specifying part 13 extracts distribution information indicating distribution close to the target distribution from the distribution information stored in the memory part 12. Here, the state specifying part 13 may extract one piece of distribution information indicating distribution closest to the target distribution. In addition, the distribution information indicating distribution closer to the target distribution may be extracted on a priority basis, to thereby extract a predetermined number of pieces of distribution information. The predetermined number is, for example, two, although not limited to any number. Moreover, with at least two pieces of distribution information as the comparison targets used to be compared with the target distribution, from among the pieces of distribution information that are the comparison targets, a piece of distribution information indicating distribution closest to the target distribution may be extracted. The distribution information extracted by the state specifying part 13, which shows distribution close to the target distribution, is hereinafter referred to as the extracted distribution information.

Here, for example, when the distribution information is extracted by the state specifying part 13, the distribution of biomolecules specified based on the distribution information may be converted into vector components. In this case, when the difference between the vector components converted from the distribution information related to the target living body and the vector components converted from the distribution information of the comparison target is small, the state specifying part 13 may extract the distribution information of the comparison target as the distribution information indicating distribution close to the target distribution.

In addition, the state specifying part 13 may learn, by machine learning, the relation between the target distribution and the distribution of biomolecules specified from the distribution information of the comparison target, and based on the result of learning, extract the distribution information indicating the distribution closer to the target distribution. In this case, for the machine learning, any method may be used.

Moreover, the state specifying part 13 may extract not only the distribution information indicating the distribution close to the target distribution, but also the distribution information indicating the distribution same as the target distribution. In addition, the state specifying part 13 may extract, for example, the distribution information indicating the distribution same or close to distribution of a part of the target distribution. In other words, the state specifying part 13 may extract other distribution information having a predetermined relation with the distribution information related to the target living body. Here, the state specifying part 13 is perceived as a specifying unit that specifies other distribution information having a predetermined relation with the distribution information related to the target living body.

The state specifying part 13 specifies a living body having biomolecules indicated in the extracted distribution information. The living body specified by the state specifying part 13 as the living body having the biomolecules indicated in the extracted distribution information is hereinafter referred to as a specified living body. Here, there are the cases where the specified living body is the target living body and the specified living body is not the target living body. The state specifying part 13 specifies a potential future state of the target living body based on the state information related to the specified living body. More specifically, as the potential future state of the target living body, the state specifying part 13 specifies the state of the specified living body at a point in time later in a time series than the point in time at which the biomolecules indicated in the extracted distribution information were collected. The state specifying part 13 creates information indicating the specified state.

In addition, based on the result of machine learning, the state specifying part 13 may estimate the future state of the target living body from the distribution information related to the target living body. More specifically, the state specifying part 13 learns the relation between the distribution of biomolecules and the state of the living body by use of the distribution information and the state information, which associate the distribution of physical quantity of biomolecules with the state of the living body after the point in time at which the distribution was obtained from the living body, as training data. The state specifying part 13 creates a learning model that takes the distribution information as input and outputs the state information based on the learning results. Then, based on the created learning model, the future state of the target living body may be estimated from the distribution information related to the target living body. Note that, for the machine learning, any method may be used.

The determination part 14, as an example of a determination unit, determines contents to be proposed related to life of the target living body. The determination part 14 uses the state information stored in the memory part 12 to extract the state information indicating a more favorable state than the recent state of the target living body specified from the state information related to the target living body, and to specify a living body that was in the state indicated in the extracted state information. Here, there are the cases where the living body specified by the determination part 14 is the target living body and is not the target living body. The determination part 14 extracts the life information related to the specified living body, and based on the contents of life specified from the extracted life information, determines the contents to be proposed related to life of the target living body. Further, the determination part 14 creates information indicating the determined contents.

Note that the determination part 14 may extract the state information indicating a more favorable state than the state specified by the state specifying part 13 as the potential future state of the target living body, and specify a living body that was in the state indicated in the extracted state information. Then, based on the life information related to the specified living body, the contents to be proposed related to life of the target living body may be determined.

In addition, based on the result of machine learning, the determination part 14 may extract the state information indicating a more favorable state than the state of the target living body from the state information related to the target living body. More specifically, the determination part 14 regards two pieces of state information, in which one state and one other state determined as a more favorable state than the one state are associated with each other, as training data, to learn the relation between two different states. Examples of the training data include: state information indicating a beauty state with a diagnosis of 40-year-old skin age; and state information indicating a beauty state with a diagnosis of 25-year-old skin age. Here, the beauty state with a diagnosis of 25-year-old skin age is an example of a more favorable state than the beauty state with a diagnosis of 40-year-old skin age. Based on the learning results, the determination part 14 creates a learning model that takes one piece of state information as input and outputs state information indicating a state that is more favorable than a state specified by the one piece of state information. Then, based on the created learning model, from the state information related to the target living body, state information indicating a state that is more favorable than a state specified by the state information related to the target living body may be extracted. Note that, for the machine learning, any method may be used.

Moreover, based on the result of machine learning, the determination part 14 may determine the contents to be proposed related to life of the target living body from the extracted state information. More specifically, the determination part 14 regards state information and life information, in which one state and life determined to contribute to the one state are associated with each other, as training data, to learn the relation between the state of the living body and the life of the living body. The training data includes: state information indicating a beauty state with a diagnosis of 25-year-old skin age; and life information indicating use of skin cream to be applied to the skin. Here, the use of skin cream is an example of life determined to contribute to the diagnosis of skin age as 25 years old. Based on the learning results, the determination part 14 may take the state information as input, extract the life information indicating the life that has contributed to a state specified by the state information, and determine the life specified by the extracted life information as the contents to be proposed related to the life of the target living body. Thus, for example, when the state information of the target living body, which indicates "the beauty state with a diagnosis of 40-year-old skin age" is inputted, the determination part 14 outputs the life information indicating "use of skin cream" as the contents to be proposed related to the life of the target living body. Note that, for the machine learning, any method may be used.

The output control part 15, as an example of a provision unit, causes the terminal 30 to output the information created by the state specifying part 13 or the determination part 14. More specifically, the output control part 15 transmits information to the terminal 30, to thereby cause the terminal 30 to display the transmitted information on the display part 31 or output the transmitted information with audio.

### <Stored contents of memory part>

Next, the contents of information stored in the memory part 12 will be described.

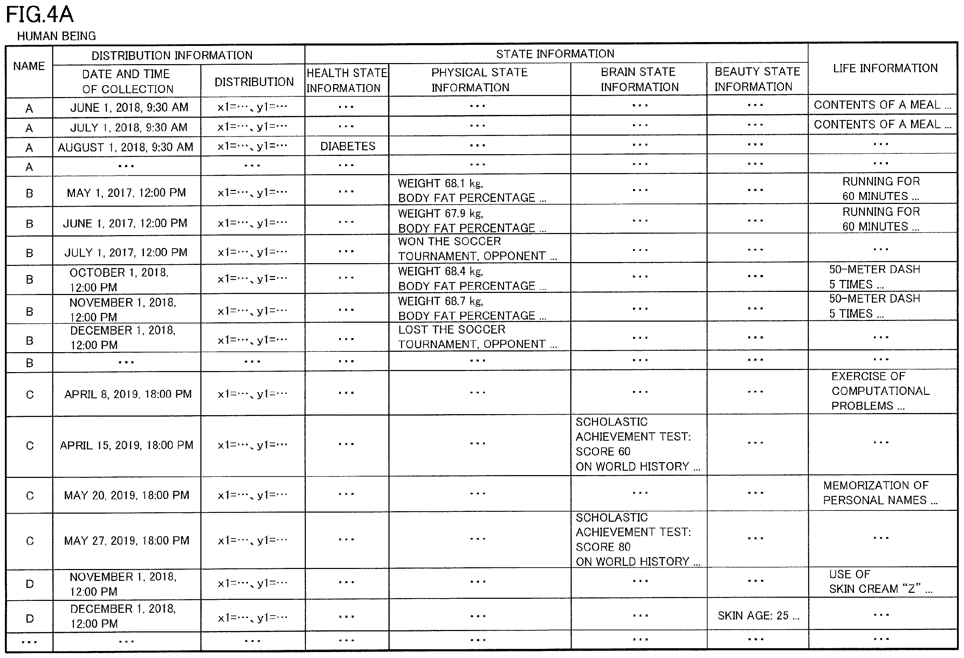

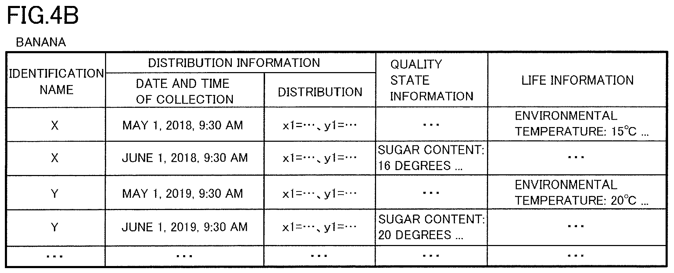

FIGS. 4A and 4B are diagrams showing examples of a living body information management table. The living body information management table is a table for managing information about a living body. The living body information management table is provided for each type of living body. FIG. 4A shows a living body information management table for "human being," which is one type of animal, and FIG. 4B shows a living body information management table for "banana," which is one type of agricultural crop.

In the living body information management table shown in FIG. 4A, the "name" indicates a name of a living body.

In addition, the "distribution information" shows "date and time of collection" and "distribution" as information included in the distribution information. The "date and time of collection" indicates the date and time when the biomolecules specified based on the distribution information were collected from the living body. In addition, the "distribution" indicates the distribution of physical quantity of biomolecules specified based on the distribution information.

Moreover, the "state information" shows "health state information," "physical state information," "brain state information," and "beauty state information."

The "health state information" indicates information about the health state of the living body. The "physical state information" indicates information about the physical state of the living body. The "brain state information" indicates information about the brain state of the living body. The "beauty state information" indicates information about the beauty state of the living body. Note that the "state information" may also indicate information about the state of growth of the living body.

In addition, the "life information" indicates, for example, information about life of the living body.

Note that the "distribution information," the "state information," and the "life information" were each transmitted from the information obtaining part 11 to the memory part 12.

In addition, the memory part 12 may also store information created by the state specifying part 13 or the determination part 14.

Here, the "physical state information" for "B" indicates the results of the games in which "B" participated, such as "won the soccer tournament" and "lost the soccer tournament." In addition, the "brain state information" for "C" indicates the results of the tests that "C" took, such as "scholastic achievement test: score 60 on world history" and "scholastic achievement test: score 80 on world history." The "state" of the living body also includes "state that has obtained a particular result" like this.

In the living body information management table shown in FIG. 4B, the "identification name" indicates a name for identifying a living body. In the example shown in the figure, the "identification name" indicates a name that discerns each of "bananas."

In addition, similar to the example in FIG. 4A, the "distribution information" shows "date and time of collection" and "distribution."

The "quality state information" indicates information about the quality state of the living body. In the example shown in the figure, as the information about the quality state of the living body, the "sugar content" of the "banana" is indicated.

Though the description is omitted in FIGS. 4A and 4B, the living body information management table is provided for each type of living body in the memory part 12.

### <State specifying processing>

Next, the flow of the state specifying processing will be described. The state specifying processing is the processing in which the server device 10 specifies the potential future state of the living body.

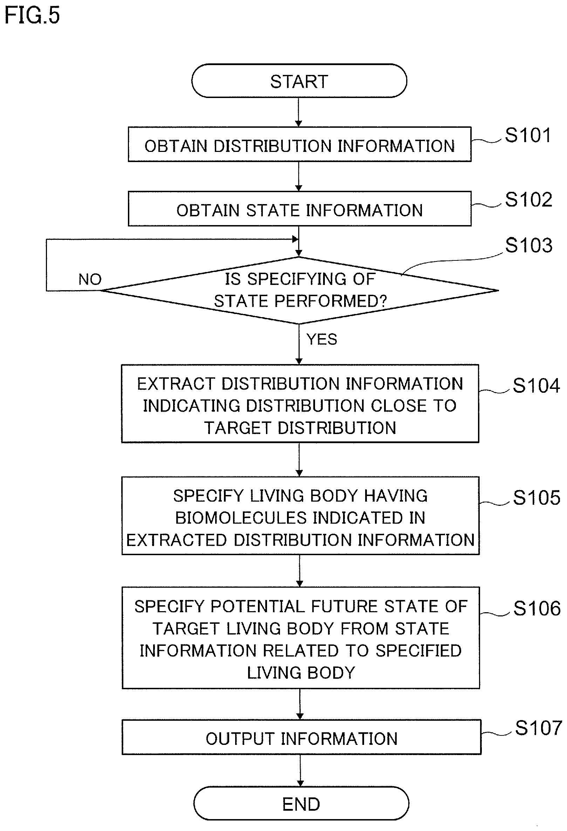

FIG. 5 is a flowchart showing a flow of the state specifying processing.

The information obtaining part 11 obtains the distribution information from the biomolecule analysis device 20 (S101).

The information obtaining part 11 also obtains the state information from, for example, the terminal 30 (S102). The distribution information and the state information obtained by the information obtaining part 11 are stored in the memory part 12.

The state specifying part 13 determines whether or not to perform specifying of the potential future state of the living body (S103). While the negative results continue, the state specifying part 13 repeats the determination operation in step 103. On the other hand, for example, when the state specifying part 13 receives an instruction to perform specifying of a potential future state of one living body from the terminal 30, obtains a positive result, and proceeds to step 104. The instruction from the terminal 30 is issued by, for example, making the instruction by use of the terminal 30 by the user thereof to perform specifying of a potential future state of one living body. In addition, for example, the state specifying part 13 may determine to perform specifying of the potential future state of the living body at a predetermined period, such as every month.

The state specifying part 13 extracts the distribution information indicating a distribution close to the target distribution (S104).

The state specifying part 13 specifies a living body having biomolecules indicated in the extracted distribution information (S105).

The state specifying part 13 also specifies a potential future state of the target living body based on the state information related to the specified living body (S106). In addition, information about the specified state is created.

The output control part 15 causes the terminal 30 to output the information created by the state specifying part 13 (S107).

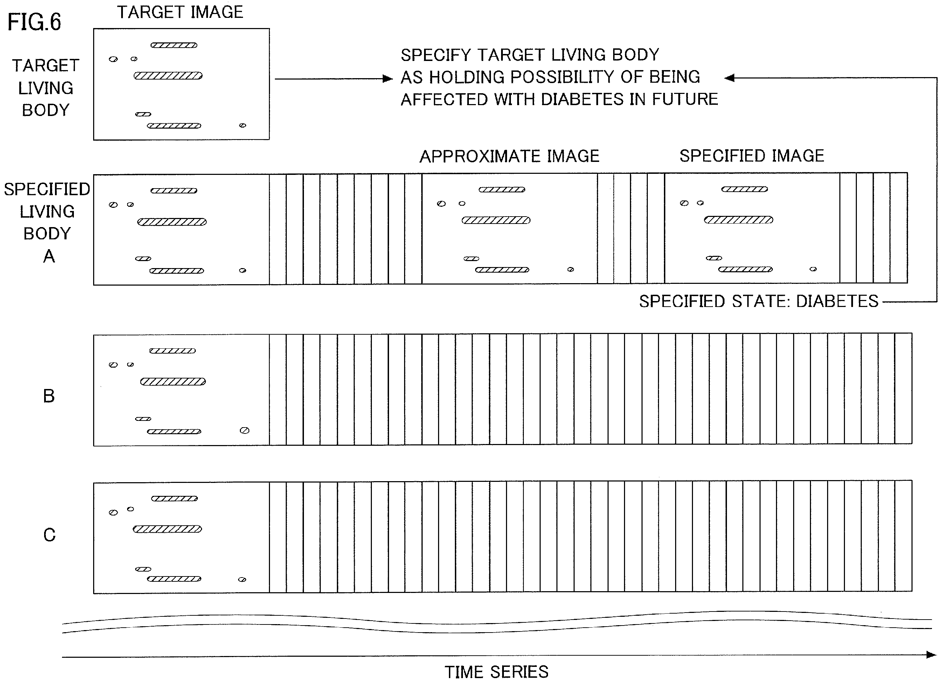

FIG. 6 is a diagram showing an example of specifying of a state by the state specifying processing (refer to FIG. 5). Each image shown in FIG. 6 is a two-dimensional electrophoresis image.

FIG. 6 shows a two-dimensional electrophoresis image related to a target living body. FIG. 6 also shows two-dimensional electrophoresis images for respective living bodies in time series. In addition, though the illustration is omitted, each two-dimensional electrophoresis image is associated with state information. These two-dimensional electrophoresis images and state information are the information accumulated in the memory part 12. Moreover, the "target image" shown in FIG. 6 is a two-dimensional electrophoresis image indicating a target distribution.

The state specifying part 13 extracts, for example, a two-dimensional electrophoresis image related to the living body "A" as a two-dimensional electrophoresis image showing a distribution close to the distribution of biomolecules specified from the target image. The two-dimensional electrophoresis image extracted by the state specifying part 13 as the two-dimensional electrophoresis image showing the distribution close to the distribution of biomolecules specified from the target image is hereinafter referred to as an approximate image. The approximate image is an example of the extracted distribution information.

The state specifying part 13 specifies a living body "A" having biomolecules shown in the approximate image.

When the state information used for specifying the potential future state of the target living body is to be extracted, the state specifying part 13 regards the state information, which indicates the state of a specified living body "A" at the point in time later in time series than the point in time at which the biomolecules shown in an approximate image have been collected from the specified living body "A," as the target of extraction. In the example shown in the figure, the state specifying part 13 regards the state information, which is associated with, from among two-dimensional electrophoresis images related to the specified living body "A," two-dimensional electrophoresis images on the right side of the approximate image, as the target of extraction. Then, the state specifying part 13 extracts the state information indicating the state of "diabetes" and specifies the "diabetes" indicated in the extracted state information as a potential future state of the target living body. Note that the state specified by the state specifying part 13 as the potential future state of the target living body is not limited to the very state indicated in the state information extracted by the state specifying part 13. The state specifying part 13 may specify the potential future state of the target living body based on the extracted state information.

Moreover, the state specifying part 13 creates information indicating the state specified as the potential future state of the target living body. The output control part 15 then causes, for example, the display part 31 of the terminal 30 to display the information created by the state specifying part 13. For example, the output control part 15 causes the display part 31 of the terminal 30 to display the message "the target living body holds the possibility of being affected with diabetes in the future," which indicates the potential future state of the target living body. In addition, the output control part 15 may display, for example, a message "a living body having biomolecules with a distribution close to the distribution of biomolecules in the target living body was affected with diabetes afterward." In other words, the information created by the state specifying part 13 may be the information based on the state information related to the specified living body, and not limited to the information indicating the potential future state of the target living body.

Note that the state specified by the state specifying part 13 as the potential future state of the target living body is hereinafter referred to as a specified state. In addition, the two-dimensional electrophoresis image associated with state information indicating the specified state is hereinafter referred to as a specified image. In the example shown in the figure, the two-dimensional electrophoresis image associated with the state information indicating "diabetes" is the specified image. Moreover, "diabetes" indicated in the state information associated with the specified image is the specified state.

In addition, in the example shown in the figure, the state specifying part 13 specified the possible future health state of the target living body, but the state to be specified is not limited thereto. The state specifying part 13 may specify, for example, the possible future beauty state of the target living body, or the possible future physical state of the target living body. Moreover, the state specifying part 13 may specify the possible future brain state of the target living body, the possible future quality state of the target living body, or the possible future growth state of the target living body.

Plural states may also be specified as the potential future state of the target living body. For example, the state specifying part 13 may specify the possible future health state of the target living body, the possible future physical state of the target living body, and the possible future brain state of the target living body by one operation of state specifying processing.

Thus, in the exemplary embodiment, from the distribution of biomolecules in the target living body, based on the state of the specified living body having biomolecules with a distribution close to the distribution, the state specifying part 13 discovers signs of the potential future state of the target living body. In other words, the state specifying part 13 estimates the future state of the target living body based on the distribution information related to the target living body, other distribution information different from the distribution information, and the state information related to the specified living body having biomolecules with distribution specified from the other distribution information.

In this case, based on the state of the specified living body, the future state of the target living body is estimated.

Moreover, in the exemplary embodiment, the state specifying part 13 specifies, in the information obtained by the information obtaining part 11, other distribution information having a predetermined relation with the distribution information related to the target living body. Then, the output control part 15 provides information based on the state of the specified living body after the point in time at which the biomolecules in the specified living body having biomolecules with distribution related to the other distribution information specified by the state specifying part 13 was with the distribution.

In addition, in the exemplary embodiment, based on the fact that the distribution of physical quantity of biomolecules is changed in accordance with the state of the living body, the distribution information for each state of the living body is covered. In this way, based on the target distribution, signs of the potential future state of the target living body are discovered.

### <Performed state specifying processing>

Next, the flow of the performed state specifying processing will be described. The performed state specifying processing is the processing in which the server device 10 specifies the potential future state of the target living body in the case where the contents determined as a proposal about life of the target living body are performed. The performed state specifying processing may be carried out by, for example, making instruction by use of the terminal 30 by the user thereof to perform the performed state specifying processing. In addition, the performed state specifying processing may be carried out, for example, by completion of the state specifying processing (refer to FIG. 5).

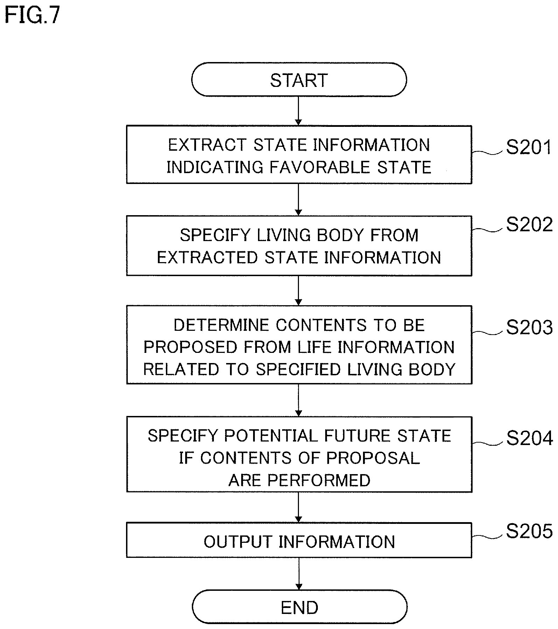

FIG. 7 is a flowchart showing the flow of the performed state specifying processing.

First, the determination part 14 extracts state information indicating a state more favorable than the state of the target living body specified by the state specifying processing (S201).

The determination part 14 specifies a living body that was in the state indicated in the extracted state information (S202).

The determination part 14 extracts life information related to the specified living body, and based on the extracted life information, determines the contents to be proposed related to life of the target living body (S203). In addition, the determination part 14 creates information about the determined contents of proposal.

The state specifying part 13 specifies a potential future state of the target living body in the case where the contents of proposal determined by the determination part 14 are performed (S204). More specifically, based on the state extracted by the determination part as a state more favorable than the state of the target living body specified by the state specifying processing, the state specifying part 13 specifies the potential future state of the target living body in the case where the contents of proposal are performed. In addition, the state specifying part 13 creates information about the specified state.

The output control part 15 causes the terminal 30 to output the information created by the determination part 14 and the information created by the state specifying part 13 (S205).

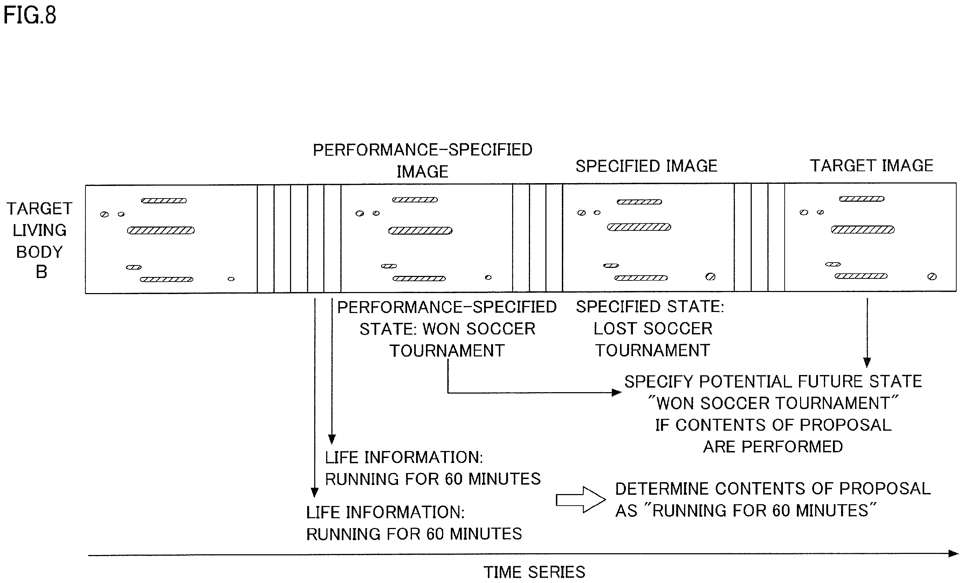

FIG. 8 is a diagram showing an example of specifying of a state by the performed state specifying processing (refer to FIG. 7). FIG. 8 shows two-dimensional electrophoresis images related to the target living body "B" (refer to FIG. 4) in time series.

First, the potential future state of the target living body is specified by the state specifying processing (refer to FIG. 5). In the example shown in the figure, the state of "lost the soccer tournament" (refer to FIG. 4) is specified as the potential future state of the target living body "B" by the state specifying processing. Note that, in the example shown in the figure, as the potential future state of the target living body, a state that the target living body itself experienced in the past is specified.

Next, in the performed state specifying processing, the determination part 14 extracts the state information indicating "won the soccer tournament," which is a more favorable state than "lost the soccer tournament" specified by the state specifying processing. Moreover, the determination part 14 specifies the target living body "B" as the living body that was in the state specified from the extracted state information. Further, the determination part 14 extracts life information indicating "running for 60 minutes" (refer to FIG. 4), which is the life of the target living body "B" at the point in time earlier in the time series than the point in time of the state "won the soccer tournament" specified from the state information extracted from among the life information related to the specified target living body "B." Then, based on the "running for 60 minutes" specified from the extracted life information, the contents to be proposed related to the life of the target living body are determined. Here, as the contents to be proposed, the determination part 14 may determine the "running for 60 minutes" itself specified from the extracted life information, or based on the extracted life information, contents that are different from those specified from the life information.

In addition, the determination part 14 creates information indicating the determined contents of proposal. The output control part 15 then causes, for example, the display part 31 of the terminal 30 to display the information created by the determination part 14. For example, the output control part 15 causes the display part 31 of the terminal 30 to display a message "We propose to the target living body to run for 60 minutes as training to achieve a more favorable physical state of the target living body," which indicates the determined contents of proposal. The output control part 15 may also display, for example, a message such as "the target living body was running for 60 minutes when it was in a more favorable state," which indicates life that the living body had, who was in the more favorable state.

Further, the state specifying part 13 specifies the potential future state of the target living body "B" in the case where the contents of proposal determined by the determination part 14 are performed on the target living body "B" as the state "won the soccer tournament." The specified state is the state extracted by the determination part 14 as the state more favorable than the state that was specified by the state specifying processing. Note that the state specifying part 13 may specify the potential future state of the target living body based on the state extracted by the determination part 14, and the potential future state of the target living body is not limited to the state extracted by the determination part 14 itself.

In addition, the state specifying part 13 creates information indicating the potential future state of the target living body in the case where the contents of proposal determined by the determination part 14 are performed. The output control part 15 then causes, for example, the display part 31 of the terminal 30 to display the information created by the state specifying part 13. For example, the output control part 15 causes the display part 31 of the terminal 30 to display the message "the target living body holds the possibility of having a state of winning a soccer tournament in the case where the proposed running for 60 minutes is carried out," which indicates the potential future state of the target living body in the case where the contents of proposal determined by the determination part 14 are performed. In addition, the output control part 15 may display, for example, a message indicating a later state of a living body who performed the contents of proposal, such as "a living body who had carried out running for 60 minutes won the soccer tournament afterwards."

Note that the state specified by the state specifying part 13 as the potential future state of the target living body in the case where the contents of proposal determined by the determination part 14 are performed is hereinafter referred to as a performance-specified state. In addition, the two-dimensional electrophoresis image associated with state information indicating the performance-specified state is hereinafter referred to as a performance-specified image. In the example shown in the figure, the two-dimensional electrophoresis image associated with the state information indicating "won the soccer tournament" is the performance-specified image. Moreover, "won the soccer tournament" indicated in the state information associated with the performance-specified image is the performance-specified state.

In addition, in the example shown in the figure, the determination part 14 determined the contents of training as the proposal for life of the target living body, but the contents of proposal is not limited thereto. For example, the determination part 14 may determine the environment in which a living body lives as the contents of proposal, or the contents about sleeping of a living body as the contents of proposal. Moreover, the determination part 14 may determine meals and nutrition that a living body takes as the contents of proposal, or products and services that a living body uses as the contents of proposal. Plural contents of proposal may also be determined.

As described above, in the exemplary embodiment, the determination part 14 determines the contents of life of a target living body proposed to have a state more favorable than the state of the target living body specified by the state specifying part 13 or the recent state of the target living body. To put it another way, the determination part 14 determines the contents to be proposed related to life of the target living body based on the state of the target living body estimated by the state specifying part 13 and the life information obtained by the information obtaining part 11.

In this case, based on the state estimated by the state specifying part 13 and the contents of life lived by the living body, the contents to be proposed related to life of the target living body are determined.

In addition, in the exemplary embodiment, the state specifying part 13 specifies effects that possibly occur to the target living body in the case where the contents of proposal determined by the determination part 14 are taken. To put it another way, the state specifying part 13 estimates a potential future state of the target living body in the case where the contents determined by the determination part 14 are performed.

In this case, for example, based on the estimated future state of the target living body in the case where the contents determined by the determination part 14 are assumed to be performed, it is determined whether or not the contents determined by the determination part 14 are adopted.

Moreover, in the exemplary embodiment, the state specifying part 13 specifies any of the states that the target living body experienced in the past as the potential future state of the target living body. To put it another way, in accordance with the distribution information extracted as another piece of distribution information from among the plural pieces of distribution information, the state specifying part 13 extracts some piece of state information from among the plural pieces of state information, and estimates the future state of the target living body from the extracted state information.

In this case, based on the past state of the target living body according to the distribution information extracted as another piece of distribution information, the future state of the target living body is estimated.

### <Time specifying processing>

Next, the flow of the time specifying processing will be described. The time specifying processing is the processing in which the server device 10 specifies time when the target living body reaches the state specified by the state specifying processing. The time specifying processing may be carried out by, for example, making instruction by use of the terminal 30 by the user thereof to perform the time specifying processing. In addition, the time specifying processing may be carried out, for example, by completion of the state specifying processing (refer to FIG. 5). Moreover, time specifying processing of the exemplary embodiment is executed in the case where plural pieces of extracted distribution information are present.

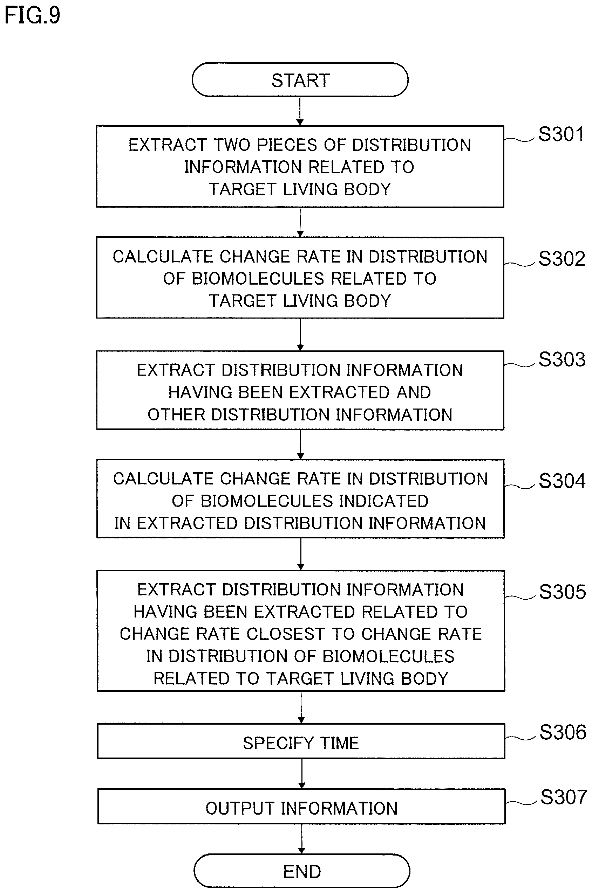

FIG. 9 is a flowchart showing a flow of the time specifying processing.

The state specifying part 13 extracts two pieces of distribution information related to the target living body (S301). More specifically, the state specifying part 13 extracts a piece of distribution information indicating the target distribution, and another piece of distribution information different from the piece of distribution information.

The state specifying part 13 calculates the change rate in the distribution of biomolecules related to the target living body from the two pieces of distribution information extracted related to the target living body (S302) .

For each of plural pieces of extracted distribution information, the state specifying part 13 extracts a piece of extracted distribution information and another piece of distribution information different from the piece of extracted distribution information (S303). Here, "another piece of distribution information" is distribution information related to a living body having biomolecules specified by the piece of extracted distribution information.

The state specifying part 13 calculates the change rate in the distribution of biomolecules indicated in a piece of extracted distribution information for each of the plural pieces of extracted distribution information (S304). More specifically, based on the piece of extracted distribution information and another piece of distribution information extracted in step 303, the state specifying part 13 calculates the change rate in the distribution of biomolecules indicated in these pieces of distribution information.

The state specifying part 13 extracts the piece of extracted distribution information related to the change rate, from among the change rate calculated for each of the plural pieces of extracted distribution information, closest to the change rate in the distribution of biomolecules related to the target living body (S305).

Based on the period during which the living body related to the piece of extracted distribution information extracted in step 305 reaches the specified state from the state indicated in the state information associated with the piece of extracted distribution information, the state specifying part 13 specifies the time when the target living body reaches the state specified by the state specifying processing (S306). In addition, the state specifying part 13 creates information about the specified time.

The output control part 15 causes the terminal 30 to output the information created by the state specifying part 13 (S307).

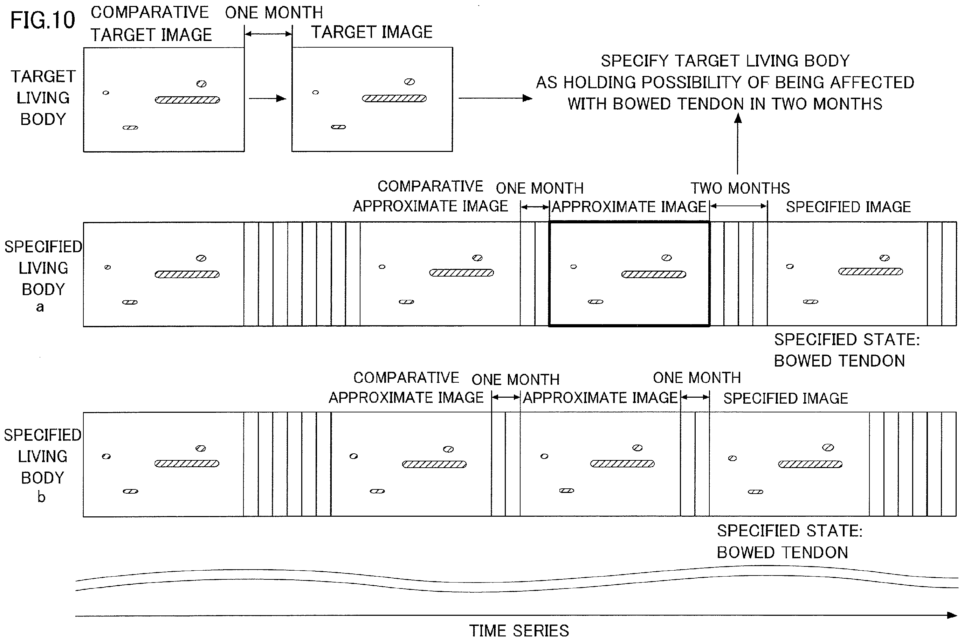

FIG. 10 is a diagram showing an example of specifying of time by the time specifying processing (refer to FIG. 9). FIG. 10 shows, as the two pieces of distribution information related to the target living body, a target image and a two-dimensional electrophoresis image different from the target image. The two-dimensional electrophoresis image different from the target image is hereinafter referred to as a comparative target image. FIG. 10 also shows two-dimensional electrophoresis images related to the specified living body "a" and two-dimensional electrophoresis images related to the specified living body "b" in a time series.

First, the potential future state of the target living body is specified by the state specifying processing (refer to FIG. 5). In the example shown in the figure, the target living body is specified to hold the possibility of being affected with "bowed tendon" in the future. Note that bowed tendon is a disease that occurs at the legs of a racehorse as an example of a living body. The "bowed tendon" is a state that both specified living body "a" and specified living body "b" have been affected in the past. In addition, regarding the specified living body "a," the period from the state related to the approximate image, namely, the state with the distribution close to the distribution of biomolecules in the target living body until the specified living body was affected with bowed tendon was two months. In contrast thereto, regarding the specified living body "b," the period from the state related to the approximate image until being affected with bowed tendon was one month.

The state specifying part 13 extracts two two-dimensional electrophoresis images for calculating the change rate in the distribution of biomolecules for each of the specified living body "a" and the specified living body "b". More specifically, for each of the specified living body "a" and the specified living body "b," the state specifying part 13 extracts the approximate image and another two-dimensional electrophoresis image different from the approximate image. The two-dimensional electrophoresis image extracted by the state specifying part 13 as another two-dimensional electrophoresis image different from the approximate image is hereinafter referred to as a comparative approximate image. Here, the period from the point in time at which the biomolecules shown in the comparative target image related to the target living body were obtained from the target living body to the point in time at which the biomolecules shown in the target image were obtained from the target living body is one month. In this case, the state specifying part 13 extracts the comparative approximate image so that the period from the point in time at which the biomolecules shown in the comparative approximate image were obtained from the specified living body to point in the time at which the biomolecules shown in the approximate image were obtained from the specified living body is close to one month, which is the period in the target living body.

The state specifying part 13 calculates the change rate in the distribution of biomolecules in the target living body from the comparative target image and the target image. In addition, for each of the specified living body "a" and the specified living body "b," the state specifying part 13 calculates the change rate in the distribution of biomolecules from the comparative approximate image and the approximate image. Then, the approximate image related to the change rate, from among the change rates calculated for the respective specified living bodies "a" and "b," close to the change rate in the distribution of biomolecules in the target living body is extracted. In the example shown in the figure, the state specifying part 13 determines that the change rate in the distribution of biomolecules in the specified living body "a" is closer to the change rate in the distribution of biomolecules in the target living body than the change rate in the distribution of biomolecules in the specified living body "b." Therefore, the state specifying part 13 extracts the approximate image related to the specified living body "a." Then, based on the period of "two months" from the state related to the approximate image of the specified living body "a" to the time when the specified living body "a" was affected with "bowed tendon," the time when the target living body will possibly be affected with "bowed tendon" in the future is specified. Here, the state specifying part 13 may specify the period between the state of the target living body related to the target image and the time being affected with "bowed tendon" as "two months" corresponding to the case of the specified living body "a," or may specify a period different from the "two months" based on the "two months" corresponding to the case of the specified living body "a."

The state specifying part 13 creates information indicating the specified time. The output control part 15 then causes, for example, the display part 31 of the terminal 30 to display the information created by the state specifying part 13. For example, the output control part 15 causes the display part 31 of the terminal 30 to display the message "the target living body holds the possibility of being affected with bowed tendon in two months," which indicates the time specified by the state specifying part 13. In addition, the output control part 15 may display, for example, the message "the specified living body with the change rate close to the change rate in the distribution of biomolecules in the target living body was affected with bowed tendon two months after the same state as the target living body," which shows the result in the specified living body.

In this manner, in the exemplary embodiment, the state specifying part 13 estimates the time when the target living body will reach a particular state. More specifically, the state specifying part 13 estimates the time based on the changes in the distribution of biomolecules specified from at least two pieces of distribution information from among the plural pieces of distribution information related to the target living body.

In this case, in accordance with the changes in the distribution of biomolecules in the target living body, the time when the target living body will reach the specified state is estimated.

Note that, in the state specifying processing, there is a case where one approximate image is extracted. In this case, based on the period during which the living body related to one approximate image reaches the specified state from the state related to the approximate image, without calculating the change rate in the distribution of biomolecules, the state specifying part 13 may specify the time when the target living body will reach the specified state in the future.

In addition, based on the result of machine learning, the state specifying part 13 may estimate the time when the target living body will reach the specified state from the distribution information related to the target living body. More specifically, the state specifying part 13 learns a relation between the distribution of biomolecules and the time when the living body reaches a particular state by use of the distribution information and the state information, which associate the distribution of physical quantities of biomolecules with time when the living body is in the particular state after the point in time at which the distribution was obtained from the living body, as training data. Based on the learning results, the state specifying part 13 creates a learning model that takes the distribution information as input, and outputs information about the time when the target living body reaches a particular state. Then, based on the created learning model, the time when the target living body reaches the specified state may be estimated from the distribution information related to the target living body. Note that, for the machine learning, any method may be used.

In the example shown in the figure, the change rate in the distribution of biomolecules of the target living body is calculated from two pieces of distribution information; however, three or more pieces of distribution information may be used for the calculation of the change rate in the distribution of biomolecules,

In addition, in the example shown in the figure, the state specifying part 13 specified the time when the target living body reaches a particular health state, but the time to be specified is not limited thereto. The state specifying part 13 may specify, for example, the time when the target living body reaches a particular beauty state, or the time when the target living body reaches a particular physical state. Moreover, the state specifying part 13 may specify the time when the target living body reaches a particular brain state, the time when the target living body reaches a particular quality state, or the time when the target living body reaches a particular growth state.

So far, the exemplary embodiment according to the present invention has been described, but the technical scope of the present invention is not limited to the scope described in the above-described exemplary embodiment. It is obvious from the following claims that various modifications and improvements added to the above-described exemplary embodiment are also included in the technical scope of the present invention.

In the exemplary embodiment, the configuration in which the living body state specifying system 1 provided with the server device 10 and the biomolecule analysis device 20 was described, but the configuration is not limited thereto.

For example, a device that combines the server device 10 and the biomolecule analysis device 20 may be provided in the living body state specifying system 1. In other words, analysis of biomolecules, creation of the distribution information, and specifying of potential future state of a living body may be performed by using one device provided in the living body state specifying system 1.

In addition, the user of the living body state specifying system 1 may be capable of setting the type of the state to be specified by the state specifying part 13 as the potential future state of the target living body. For example, it may be possible for the user to set, by the operation of the terminal 30, which of the following states is to be specified by the state specifying part 13 as the potential future state of the target living body: the health state; the state about beauty of the living body; the physical state of the living body; the brain state of the living body; the quality state of the living body; and the growth state of the living body. Then, the state specifying part 13 may specify the state of the type instructed by the terminal 30 as the potential future state of the target living body.

In addition, the user of the living body state specifying system 1 may be capable of setting the type of the proposal, which concerns the life of the target living body, to be determined by the determination part 14. For example, it may be possible for the user to set, by the operation of the terminal 30, which of the following proposals is to be determined by the determination part 14 as the proposal about the life of the target living body: a proposal about the environment in a place where the living body lives; a proposal about sleep of the living body; a proposal about exercise or training that the living body performs; a proposal about meals and nutrition that the living body takes; and a proposal about products and services that the living body adopts. Then, the determination part 14 may determine the proposal of the type instructed by the terminal 30 as the proposal about the life of the target living body.

In addition, in the exemplary embodiment, it was described that the distribution information, the state information, and the life information were associated with one another and stored in the memory part 12, but not limited thereto.

The distribution information, the state information, and the life information may be stored in the memory part 12 without being associated with one another. In this case, each distribution information should include information about the time when the biomolecules indicated in the distribution information were collected from the living body. In addition, each state information should include information about the time when the state of the living body indicated in the state information was present. Moreover, each living body information should include information about the time when the life of the living body indicated in the life information was lived. In other words, the distribution information, the state information, and the life information should be arranged in a time series.

In addition, in the exemplary embodiment, it was described that the user operated the terminal 30 or the server device 10 to input the state information or the life information, but not limited thereto.

For example, a living body may be photographed using means for photographing, such as a video camera. In addition, the living body shown on the video taken may be analyzed by using analysis methods for analyzing living bodies, the state information indicating the state of the living body or the life information indicating the life of the living body may be created from the analysis results, and the created state information or life information may be transmitted to the information obtaining part 11. In this manner, the information obtaining part 11 may obtain the state information or the life information by way of functional means different from the terminal 30.

Moreover, in the exemplary embodiment, the determination part 14 determines the contents of proposal about the life of the target living body to have more favorable state than the state specified by the state specifying part 13, but not limited thereto.

For example, the determination part 14 may determine the contents of proposal about the life of the target living body not to have less favorable state than the state specified by the state specifying part 13. For example, in the case where it is specified by the state specifying part 13 that the target living body will possibly have the state "score 80 on world history" (refer to FIG. 4A), the determination part 14 may determine the contents of proposal not to have the state "score 60 on world history," which is less favorable than "score 80 on world history." More specifically, the determination part 14 may determine, as the contents of proposal about the life of the target living body, that the living body "D" does not do "exercise of computational problems," which was the life at the point in time earlier in the time series than the point in time of "score 60 on world history." In this case, for example, the determination part 14 may cause the display part 31 of the terminal 30, via the output control part 15, to display the message "we propose not to spend the learning time on the exercise of computational problems to prevent the target living body from reaching the unfavorable state," which indicates the contents of proposal.

In addition, the exemplary embodiment had the configuration in which the server device 10 specified the potential future state of the living body, but the configuration is not limited thereto.

For example, the terminal 30 may have the function of the server device 10. In other words, the terminal 30 may have the functions of the information obtaining part 11, the memory part 12, the state specifying part 13, the determination part 14, and the output control part 15, etc. of the server device 10.

In addition, the program for implementing the exemplary embodiment of the present invention can be provided in the state of being stored in a computer-readable recording medium, such as a magnetic recording medium(a magnetic tape, a magnetic disk, etc.), an optical recording medium (an optical disk, etc.), or a semiconductor memory. Moreover, the program can be provided by use of communication means such as the Internet, etc.

### Reference Signs List

1 Living body state specifying system
10 Server
11 Information obtaining part
12 Memory part
13 State specifying part
14 Determination part
15 Output control part
20 Biomolecule analysis device
30 Terminal

## Claims

1. An information processing system comprising:
an obtaining unit obtaining state information about a state of a living body and distribution information about a distribution of physical quantity of biomolecules; and
an estimation unit estimating a future state of one living body based on the distribution information related to the one living body, other distribution information different from the distribution information, and the state information related to a particular living body having biomolecules with the distribution specified from the other distribution information.

2. The information processing system according to claim 1, further comprising:
a life information obtaining unit obtaining life information related to life of a living body; and
a determination unit determining contents to be proposed related to life of the one living body based on the state of the one living body estimated by the estimation unit and the life information obtained by the life information obtaining unit.

3. The information processing system according to claim 2, wherein the estimation unit estimates a future state of the one living body if the contents determined by the determination unit are performed.

4. The information processing system according to claim 1, wherein the estimation unit estimates time when the one living body reaches the state.

5. The information processing system according to claim 4, wherein
the obtaining unit obtains a plurality of pieces of distribution information about the distribution at different points in time for the one living body, and
the estimation unit estimates the time based on change in the distribution of biomolecules specified from at least two pieces of distribution information from among the plurality of pieces of distribution information.

6. The information processing system according to claim 1, wherein
the obtaining unit obtains a plurality of pieces of distribution information about the distribution at different points in time for the one living body, and a plurality of pieces of state information about a state of the one living body at different points in time,
the other distribution information is the distribution information related to the one living body, and
the estimation unit extracts, in accordance with the distribution information extracted as the other distribution information from among the plurality of pieces of distribution information, some of the pieces of state information from among the plurality of pieces of state information, and estimates a future state of the one living body by the extracted state information.

7. An information processing system comprising:
an obtaining unit obtaining distribution information about a distribution of physical quantity of biomolecules in a living body;
a specifying unit specifying, in the information obtained by the obtaining unit, other distribution information having a predetermined relation with the distribution information related to one living body; and
a provision unit providing information based on a state of a particular living body after a point in time at which biomolecules in the particular living body having biomolecules with the distribution related to the other distribution information specified by the specifying unit showed the distribution.

8. A program causing a computer to implement:
an obtaining function obtaining state information about a state of a living body and distribution information about a distribution of physical quantity of biomolecules; and
an estimation function estimating a future state of one living body based on the distribution information related to the one living body, other distribution information different from the distribution information, and the state information related to a particular living body having biomolecules with the distribution specified from the other distribution information.
